# EUROPÄISCHE PATENTSCHRIFT

(11) **EP 1 258 200 B1**
(45) Veröffentlichungstag und Bekanntmachung des Hinweises auf die Patenterteilung: **16.02.2005**
(21) Anmeldenummer: 02009275.5
(22) Anmeldetag: 29.04.2002
(51) Int. Cl.: A23L 1/226, A61K 47/22, C07D 311/30, C07D 311/32

(54) **Verwendung von Hydroxyflavanone zur Maskierung des bitteren Geschmacks, und Lebensmittel und pharmazeutische Zusammensetzungen enthaltend eine wirksame Menge dieser Hydroxyflavone**
Use of hydroxyflavones for masking of bitterness, and food and pharmaceutical compositions comprising an effective amount thereof
Utilisation des hydroxyflavones pour cacher le goût amer, et compositions alimentaires et pharmaceutiques comprenant une quantité effective de ces hydroxyflavones

(30) Priorität: 11.05.2001 DE 10122898
(43) Veröffentlichungstag der Anmeldung: 20.11.2002
(73) Patentinhaber: Symrise GmbH & Co. KG, 37603 Holzminden (DE)
(72) Erfinder: Ley, Jakob Peter, Dr., 37603 Holzminden (DE); Krammer, Gerhard, Dr., 37603 Holzminden (DE); Kindel, Günter, 37671 Höxter (DE); Gatfield, Ian-Lucas, Dr., 37671 Höxter (DE); Müller, Manfred, 37603 Holzminden (DE)
(74) Vertreter: Eisenführ, Speiser & Partner

(56) Entgegenhaltungen:
- US-A- 5 703 053
- WILLIAM E. DICK, JR.: "Structure-Taste Correlations for Flavans and Flavanones Conformationally Equivalent to Phyllodulcin" J. AGRIC. FOOD CHEM., Bd. 29, 1981, Seiten 305-312, XP002236604

## Beschreibung

Die Erfindung betrifft die Verwendung von hydroxysubstituierten 2-Phenyl-chroman-4-onen (im Folgenden als Hydroxyflavanone bezeichnet), deren Salzen und Stereoisomeren sowie Gemischen derselben zur Maskierung oder Verminderung des bitteren und/oder metallischen Geschmackseindrucks. Fernerhin betrifft die Erfindung der Ernährung oder dem Genuss dienende oder orale pharmazeutische Zubereitungen, gekennzeichnet dadurch, dass diese einen wirksamen Gehalt an den bezeichneten Hydroxyflavanonen, deren Salzen und Stereoisomeren sowie Gemischen derselben besitzen.

Nahrungs- oder Genussmittel enthalten häufig verschiedene Bitterstoffe, die zwar einerseits charakteristisch sind (z.B. Coffein in Tee oder Kaffee), andererseits den Wert aber auch stark mindern können (z.B. Limonoide in Zitrus-Säften, bitterer Nachgeschmack vieler künstlicher Süßstoffe wie Aspartam oder Saccharin). Zur Senkung des natürlichen Gehalts an Bitterstoffen ist daher oft eine nachträgliche Behandlung nötig, beispielsweise extraktiv wie bei der Entcoffeinierung von Tee bzw. Kaffee, oder enzymatisch, z.B. Behandlung von Orangensaft mit einer Glycosidase zur Zerstörung des bitteren Naringins oder Einsatz von speziellen Peptidasen bei der Reifung von Käse. Diese Behandlung ist belastend für das Produkt, erzeugt Abfallstoffe und bedingt z.B. auch Lösungsmittelreste und andere Rückstände (Enzyme) in den Produkten. Daher ist es wünschenswert, Stoffe, bevorzugt natürliche oder naturidentische Stoffe zu finden, die den bitteren Geschmack oder Nachgeschmack wirkungsvoll unterdrücken oder zumindest vermindern können.

Besonders wichtig ist die Unterdrückung des bitteren Geschmacks bei vielen pharmazeutischen Wirkstoffen, da dadurch die Bereitschaft der Patienten, insbesondere bei bitterempfindlichen Patienten wie Kindern, die Zubereitung oral zu sich zu nehmen, deutlich erhöht werden kann. Viele pharmazeutische Wirkstoffe, beispielsweise Chinin, haben einen ausgeprägten bitteren Geschmack und/oder Nachgeschmack.

Bisher sind wenige Stoffe beschrieben worden, die die Bitterkeit unterdrücken und dabei keinen Eigengeschmack besitzen. So werden in US-A 6,083,459 als Bitter-Maskierer wirkende Aminosäurederivate beschrieben, die aber nicht in der Natur vorkommen. 2(-4-Methoxyphenoxy)propionsäure-Natriumsalz (Lactisol) zeigt einen schwachen bitter-reduzierenden Effekt bei relativ hohen Dosen (450 ppm), wie in *Chem. Senses,* 1994, Bd. 19, S. 349 ff. berichtet; problematisch ist aber, dass die Substanz gleichzeitig den süßen Geschmackseindruck unterdrückt (US-A 4,567,053). Das Neohesperidindihydrochalcon zeigt auch einen bitter-reduzierenden Effekt, ist aber vor allem ein Süßstoff (vgl. *Manufacturing Chemist* 2000, Juli-Heft, S. 16-17), der in nicht-süßen Anwendungen auch störend wirkt. In US-A 5,580,545 wird zwar für einige Flavone (2-Phenylchrom-2-en-4-one) geschmacksverändernde Eigenschaften beschrieben, eine Bitter-reduzierende oder -unterdrückende Wirkung wurde aber nicht gefunden.

Natriumchlorid zeigt einen bitter-maskierenden Effekt gegen viele Bitterstoffe (z.B. *Nature*, 1997, Bd. 387, S. 563); allerdings kann die Aufnahme größerer Mengen Salz z.B. zu Herz-Kreislauferkrankungen führen.

Eingedickte Extrakte aus Herba Santa bzw. einfache wässrige oder alkholische Auszüge aus derselben zeigen eine schwach bitter-maskierende Wirkung gegenüber Chinin; bisher wurden aber die einzelnen Inhaltsstoffe nicht auf ihre Wirkung untersucht; zudem zeigen die Extrakte einen recht starken kräuterartigen Eigengeschmack.

In WO 00/21 390 wird Polyglutaminsäure als Bitterkeit-unterdrückendes Agens beschrieben; dabei werden relativ hohe Konzentrationen im Bereich um 1 Gew.-% benötigt. Ein Lipoprotein, bestehend aus β-Lactoglobulin und Phosphatidinsäure, zeigt ebenfalls einen bitter-maskierenden Effekt (EP-A 635 218). Solche Polymere sind allerdings schwierig zu charakterisieren und zu standardisieren.

Das Flavonglycosid Neodiosmin (5,7-Dihydroxy-2-(4-methoxy-3-hydroxyphenyl)-7-O-neohesperidosyl-chrom-2-en-4-on) zeigt eine Bitter-unterdrückende Wirkung (US-A 4,154,862), zeichnet sich aber durch einen Disaccharid-Rest aus, der die Herstellung und Anwendbarkeit der Substanz erschwert.

In US-A 5,703,053 wird u.a. die Bitterkeit-unterdrückende Wirkung von 5-Hydroxyflavon beschrieben.

Aufgabe der vorliegenden Erfindung war es, einfach zugängliche niedermolekulare Stoffe zu finden, die einen Bitter-unterdrückenden Effekt gegen eine Vielzahl von Bitterstoffen zeigen.

Die Erfindung betrifft daher die Verwendung von Verbindungen der allgemeinen Formel (I) 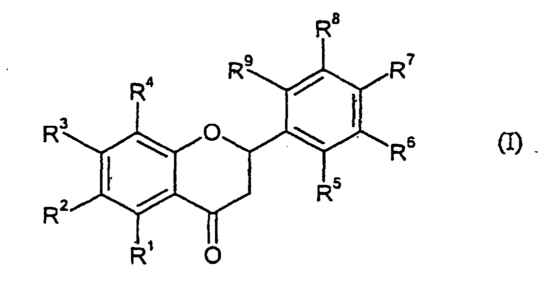 wobei
- R², R⁴, R⁵ und R⁹: Wasserstoffatome darstellen,
- R¹, R³, R⁶, R⁷ und R⁸: unabhängig voneinander Wasserstoffatome, Hydroxygruppen, Methyl-, Methoxy- oder Ethoxygruppen bedeuten, mit der Maßgabe, dass mindestens einer der Reste R⁶ bis R⁸ eine Hydroxygruppe darstellt,
als Bestandteil von in der Ernährung oder dem Genuss dienenden sowie oralen pharmazeutischen Zubereitungen, enthaltend mindestens einen Bitterstoff oder einen Stoff, der einen bitteren Nachgeschmack verursacht, zur Maskierung oder Verminderung des bitteren oder metallischen Geschmackseindrucks. Bitterstoffe im Sinne der Erfindung können beispielsweise sein: Xanthinalkaloide (z.B. Coffein, Theobromin), Chinolinderivate (z.B. Chinin), Limonoide (z.B. Limonin aus Zitrusfrüchten), Polyphenole (z.B. Catechine, Flavonole, γ-Oryzanol, Hesperitin), pharmazeutische Wirkstoffe (z.B. Fluorchinolon-Antibiotika, Aspirin, β-Lactam-Antibiotika, Ambroxol, Paracetamol, Aspirin, Guaifenesin), Denatoniumbenzoat, Sucraloseoctaacetat, Kaliumchlorid, Magnesiumsalze, Harnstoff, bittere Aminosäuren (z.B. Tryptophan) und bittere Peptidbruchstücke (z.B. mit endständigem Leucin- oder Isoleucinrest).

Stoffe, die einen bitteren Nachgeschmack im Sinne der Erfindung haben, können beispielsweise sein: Aspartam, Neotam, Saccharin und Cyclamat.

Die der Ernährung oder dem Genuss dienenden Zubereitungen im Sinne der Erfindung sind z.B. Backwaren (z.B. Brot, Trockenkekse, Kuchen, sonstiges Gebäck), Süßwaren (z.B. Schokoladen, Hart- und Weichkaramellen, Kaugummi), alkoholische oder nicht-alkoholische Getränke (z.B. Kaffee, Tee, Wein, Bier, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke, Fleischprodukte (z.B. Schinken, Wurstzubereitungen), Getreideprodukte (z.B. Frühstückscerialien, Müsliriegel), Milchprodukte (z.B. Milchgetränke, Milcheis, Joghurt, Kefir, Käse, Trockenmilchpulver, Molke), Fruchtzubereitungen (z.B. Konfitüren, Fruchteis, Fruchtsoßen), Gemüsezubereitungen (z.B. Ketchup, Soßen), Knabberartikel (z.B. frittierte Kartoffelchips, Extrudate auf Mais- oder Erdnussbasis), Produkte auf Fett- und Ölbasis oder Emulsionen derselben (z.B. Mayonnaise, Remoulade, Dressings), Gewürze, Riech-, Aroma- und Geschmacksstoffkompositionen, Würzmischungen, Fertiggerichte und Suppen. Die Zubereitungen im Sinne der Erfindung, insbesondere bevorzugt die Riech-, Aroma- und Geschmacksstoffkompositionen sowie Würzmischungen können auch als Halbfertigware zur Herstellung weiterer der Ernährung oder dem Genuss dienenden Zubereitungen dienen.

Orale pharmazeutische Zubereitungen im Sinne der Erfindung sind Zubereitungen, die z.B. in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

Besonders bevorzugt ist die Verwendung von Verbindungen der allgemeinen Formel (I), wobei
- R², R⁴, R⁵, R⁸ und R⁹: Wasserstoffatome darstellen,
- R¹, R³ und R⁶: unabhängig voneinander Wasserstoffatome, Hydroxy- oder Methoxygruppen bedeuten, mit der Maßgabe, dass mindestens einer der Reste R¹ und R³ eine Hydroxygruppe darstellt,
und
- R⁷: eine Hydroxygruppe darstellt,
in der Ernährung oder dem Genuss dienenden sowie oralen pharmazeutischen Zubereitungen, enthaltend mindestens einen Bitterstoff oder einen Stoff, der einen bitteren Nachgeschmack verursacht, zur Maskierung oder Verminderung des bitteren oder metallischen Geschmackseindrucks.

Die erfindungsgemäßen Hydroxyflavanone können bevorzugt als ein- oder - im Falle mehrerer Hydroxygruppen - mehrwertige Anionen vorliegen, wobei als Gegenkationen die einfach positiv geladenen Kationen der ersten Haupt- und Nebengruppe, das Ammoniumion, ein Trialkylammoniumion, die zweiwertig geladenen Kationen der zweiten Haupt- und Nebengruppe sowie die dreiwertigen Kationen der 3. Haupt- und Nebengruppe dienen, bevorzugt Na⁺, K⁺, NH₄⁺, Ca²⁺, Mg²⁺, Al³⁺ und Zn²⁺.

Die erfindungsgemäßen Hydroxyflavanone können als (*2S*)- oder (*2R*)-Enantiomer oder als Mischung der beiden vorliegen. Bevorzugt liegen die erfindungsgemäßen Hydroxyflavanone als (*2S*)-Enantiomer oder als an (*2S*)-Enantiomer angereichertem Gemisch vor.

Ohne die Erfindung damit einzuschränken, seien als beispielhafte Verbindungen genannt: 2-(4-Hydroxyphenyl)-5,7-dihydroxychroman-4-on (Naringenin), 2-(3,4-Dihydroxyphenyl)-5,7-dihydroxychroman-4-on (Eriodictyol), 2-(3,4-Dihydroxyphenyl)-5-hydroxy-7-methoxychroman-4-on (Eriodictyol-7-methylether), 2-(3,4-Dihydroxyphenyl)-7-hydroxy-5-methoxychroman-4-on (Eriodictyol-5-methylether) und 2-(4-Hydroxy-3-methoxyphenyl)-5,7-dihydroxychroman-4-on (Homoeriodictyol), deren (*2S*)- oder (*2R*)-Enantiomere oder Gemische derselben sowie deren ein- oder mehrwertigen Phenolatsalze mit Na⁺, K⁺, NH₄^{+,} Ca²⁺, Mg²⁺ oder Al³⁺ als Gegenkationen.

Im folgenden Schema sind die Strukturen der insbesondere bevorzugten erfindungsgemäßen Beispiele verdeutlicht:

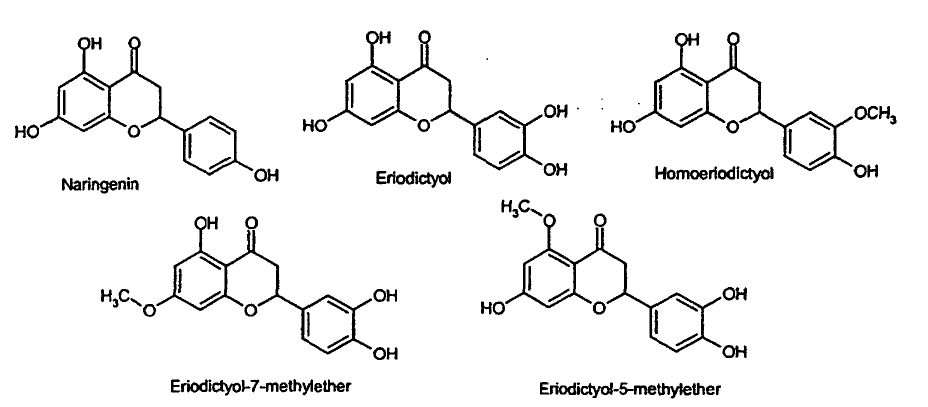

Insbesondere bevorzugt sind die Mono- und Dinatriumsalze des (+)-(2*S*)-Homoeriodictyols.

Selbstverständlich können die verschiedenen erfindungsgemäßen Hydroxyflavanone, deren Stereoisomere und Salze jeweils alleine oder als Gemische erfindungsgemäß verwendet werden.

Überraschenderweise wurde gefunden, dass die erfindungsgemäßen Hydroxyflavanone auch in sehr geringen Konzentrationen den bitteren Geschmackseindruck einer Vielzahl von Bitterstoffen, insbesondere von Methylxanthinen wie z.B. Coffein, Alkaloiden, wie z.B. Chinin, Flavonoiden wie z.B. Naringin, anorganischen Salzen wie Kaliumchlorid oder Magnesiumsulfat, pharmazeutischen Wirkstoffen wie z.B. β-Lactamantibiotika, Paracetamol, Guaifenesin reduzieren oder sogar vollständig unterdrücken können, wobei es besonders vorteilhaft ist, dass die erfindungsgemäßen Hydroxyflavanone nahezu keinen Eigengeschmack besitzen. Insbesondere sind die erfindungsgemäßen Hydroxyflavanone direkt in der Zubereitung bei der Aufnahme in den Mund wirksam, und müssen nicht zeitlich vor dem Bitterstoff aufgenommen werden.

Ein Teil der Hydroxyflavanone sind schon lange bekannt und wurden in der Natur gefunden; z.B. kommt Naringenin als Glycosid gebunden in Pampelmusen vor; Homoeriodictyol wurde als Minorkomponente aus Chryothamnus spp. (*Phytochemistry*, 1999, Band 51, Heft 6, S. 771-780) und zusammen mit Eriodictyol aus *Eriodictyon californicum* isoliert (*J. Am. Chem. Soc.* 1940, Band 62, S. 3285). Eriodictyol-7-methylether wurde aus *Dubantia arborea* isoliert (Biochem. Syst. Ecol., 1999, Bd. 27, Heft 7, S. 755-757).

Weiterer Gegenstand der Erfindung sind der Ernährung oder dem Genuss dienende oder orale pharmazeutische Zubereitungen, dadurch gekennzeichnet, dass sie einen wirksamen Gehalt an Hydroxyflavanonen, deren Salze sowie deren Gemische enthalten. Sie enthalten in der Regel 0,000001 Gew.-% bis 10 Gew.-%, bevorzugt 0,00001 bis 1 Gew.-%, insbesondere aber 0,0001 Gew.-% bis 0,1 Gew.-%, bezogen auf das Gesamtgewicht der Zubereitung, an erfindungsgemäßen Hydroxyflavanonen, deren Salzen sowie deren Gemische. Weitere übliche Wirk-, Grund-, Hilfs- und Zusatzstoffe für Nahrungs- oder Genussmittel oder orale pharmazeutische Zubereitungen können in Mengen von 5 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Die erfindungsgemäßen Zubereitungen, die als Halbfertigwaren dienen, enthalten in der Regel 0,0001 Gew.-% bis 95 Gew.-%, bevorzugt 0,1 bis 80 Gew.-%, insbesondere aber 1 Gew.-% bis 50 Gew.-%, bezogen auf das Gesamtgewicht der Zubereitung, an erfindungsgemäßen Hydroxyflavanonen, deren Salzen sowie deren Gemische.

Die erfindungsgemäßen Zubereitungen, enthaltend eins oder mehrere der erfindungsgemäßen Hydroxyflavanone, werden hergestellt, dergestalt, dass die erfindungsgemäßen Hydroxyflavanone als Substanzen, als Lösung oder in Form eines Gemischs mit einem festen oder flüssigen Trägerstoff in die der Ernährung oder dem Genuss dienenden Zubereitungen eingearbeitet werden.

Zur Herstellung der Zubereitungen können in einer weiteren Ausführungsform die erfindungsgemäßen Hydroxyflavanone und gegebenenfalls andere Bestandteile der erfindungsgemäßen Zubereitung auch vorher in Emulsionen, in Liposomen, z.B. ausgehend von Phosphatidylcholin, in Microsphären, in Nanosphären oder auch in Kapseln aus einer für Lebens- und Genussmittel geeigneten Matrix, z.B. aus Stärke, Stärkederivaten, anderen Polysacchariden, natürlichen Fetten, natürlichen Wachsen oder aus Proteinen, z.B. Gelatine, eingearbeitet werden. Eine weitere Ausflihrungsform besteht darin, dass die erfindungsgemäßen Hydroxyflavanone vorher mit geeigneten Komplexbildnern, beispielsweise mit Cyclodextrinen oder Cyclodextrinderivaten, bevorzugt β-Cyclodextrin, komplexiert werden und in dieser Form eingesetzt werden.

Als andere Bestandteile für die erfindungsgemäßen, der Ernährung oder dem Genuss dienenden Zubereitungen können weitere übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nichtproteinogene Aminosäuren und verwandte Verbindungen (z.B. Taurin), Peptide, native oder prozessierte Proteine (z.B. Gelatine), Enzyme (z.B. Peptidasen), Nukleinsäuren, Nucleotide, geschmacksmodulierende Stoffe (z.B. Natriumglutamat, Inositolphosphat, 2-Phenoxypropionsäure), Emulgatoren (z.B. Lecithine, Diacylglycerole, Monoacylglycerole), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), zusätzliche Bitterstoffe (z.B. Chinin, Coffein, Limonin), Süßstoffe (z.B. Saccharin, Cyclamat, Aspartam, Neotam), mineralische Salze (z.B. Natriumchlorid, Kaliumchlorid, Magnesiumchlorid, Natriumphosphate), die enzymatische Bräunung verhindernde Stoffe (z.B. Schwefeldioxid, Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, synthetische, natürliche oder naturidentische Aromastoffe oder Riechstoffe sowie Geruchskorrigentien und nicht den bitteren Geschmack betreffende Geschmackskorrigentien.

Als andere Bestandteile für die erfindungsgemäßen oralen pharmazeutischen Zubereitungen können alle üblicherweise weiteren Wirk-, Grund-, Hilfs- und Zusatzstoffe für orale pharmazeutische Zubereitungen verwendet werden. Als Wirkstoffe können alle bitter oder metallisch schmeckenden oral formulierbaren pharmazeutischen Wirkstoffe verwendet werden. Die Wirk-, Grund-, Hilfs- und Zusatzstoffe können in an sich bekannter Weise in die oralen Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geruchskorrigentien sowie nicht den bitteren Geschmack betreffende Geschmackskorrigentien.

Bevorzugt können die erfindungsgemäßen Zubereitungen auch noch eine Aromakomposition enthalten, um den Geschmack und/oder Geruch der Zubereitung abzurunden und zu verfeinern. Geeignete Aromakompositionen enthalten z.B. synthetische, natürliche oder naturidentische Aroma-, Riech- und Geschmacksstoffe sowie geeignete Hilfs- und Trägerstoffe. Als besonders vorteilhaft wird dabei angesehen, dass der bittere oder metallische Geschmackseindruck von in den erfindungsgemäßen Aromakompositionien enthaltenenen Aroma- oder Riechstoffen unterdrückt oder vermindert werden kann und damit das gesamte Aroma- oder Geschmacksprofil verbessert wird.

Ein weiterer Gegenstand der Erfindung ist die Verwendung der der Ernährung oder dem Genuss dienenden oder pharmazeutischen Zubereitungen als Halbfertigwaren zur Unterdrückung oder Reduzierung des bitteren Geschmacks oder Nachgeschmacks von daraus gefertigten Zubereitungen als Fertigwaren.

### Beispiele

Die Beispiele dienen nur zur Verdeutlichung der Erfindung, ohne diese damit einzuschränken.

### Beispiel 1: Entbitterung von Schwarztee

Zwei verschiedene Schwarzteesorten wurden mit kochendem Wasser aufgebrüht (3 g Tee auf 100 ml, 3 min ziehen lassen, filtrieren) und anschließend von einer Expertengruppe verkostet. Die Bitterkeit wurde mit einer Skala von 1 bis 5 bewertet (1 gerade wahrnehmbar bitter, 5 sehr bitter). Die Ergebnisse sind in der folgenden Tabelle wiedergegeben:

**Tabelle 1**

| **Probe** | **Bewertung (1-5)** |
|---|---|
| Teemuster 1 | 3,5 |
| Teemuster 2 | 5 |
| Teemuster 2 + 0,05 % Homoeriodictyol-Dinatriumsalz | 2 |

### Beispiel 2: Maskierung des Bittergeschmacks eines β-Lactam-Antibiotikums

Es wurden eine Lösung von Faropenem-Daloxat ([5*R*-[3(*R**),5α,6α(*R**)]]-6-(1-Hydroxyethyl)-7-oxo-3-(tetrahydro-2-furanyl)-4-thia-1-aza-bicyclo[3.2.0]hept-2-ene-2-carbonsäure-(5-methyl-2-oxo-1,3-dioxol-4-yl)methyl-ester, CAS-Nr. 141702-36-5, 0,1 Gew.-% in Wasser, Probe 1) und eine Mischung von Faropenem-Daloxat + Homoeriodictyol-Dinatriumsalz (je 0,1 Gew.-%, in Wasser, Probe 2) angefertigt. Einer Gruppe von 7 Prüfern wurden jeweils 3 Proben zur Unterschiedsprüfung vorgelegt. 4 Prüfer erhielten die Probe 1 als Doppelprobe, 3 Prüfer die Probe 2 als Doppelprobe. Dabei werden die Proben in unterschiedlicher Reihenfolge verkostet.

Von 7 Prüfern haben 7 die Einzelprobe richtig erkannt. Die durchschnittliche Bitterintensität der Probe 1 wurde mit 5, die von Probe 2 mit 2 bewertet (Skala 1 bis 5).

### Beispiel 3: Bitter-Reduzierung einer Coffeinlösung durch (2S)-Homoeriodictyol-Dinatriumsalz

Um die Reduzierung des Bitter-Eindrucks zu quantifizieren, wurde die Bitterkeit einer 500 ppm enthaltenden Coffeinlösung und einer Probe, die 500 ppm Coffein und eine wechselnde Menge (*2S*)-Homoeriodictyol-Dinatriumsalz enthielt, bestimmt.

Zur Einstufung der Prüfproben wurde eine Referenzreihe mit 10 verschiedenen Konzentrationen von Coffein in Wasser (50, 100, 150, 200, 250, 300, 350, 400, 450 und 500 ppm) hergestellt. In der folgenden Abbildung ist die Abhängigkeit der Bitter-Erkennung von der zugesetzten Menge Homoeriodictyol-Dinatriumsalz zu erkennen:

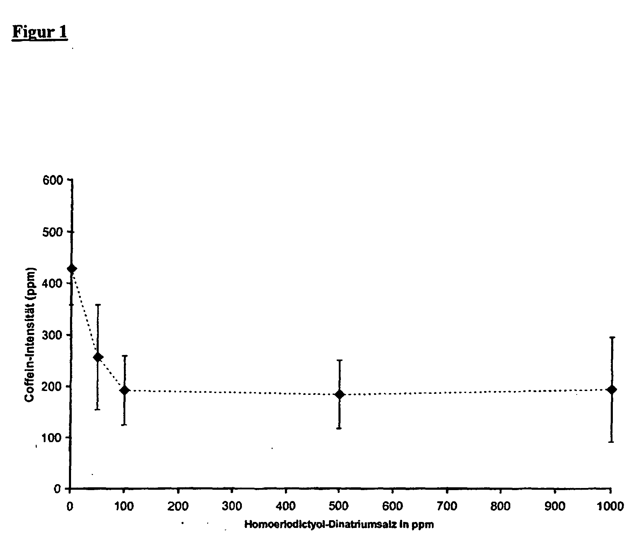

Fig. 1 zeigt den Verlauf der Bitterintensität einer 500 ppm Coffein enthaltenden Lösung bei steigender Konzentration an (*2S*)-Homoeriodictyol-Dinatriumsalzkonzentration im Vergleich zu einer Konzentrationsreihe von Coffein (100 bis 500 ppm)

### Beispiel 4: Bitter-Reduzierung einer Coffeinlösung durch verschiedene Hydroxyflavanone

Analog zu Beispiel 3 wurde eine Coffeinlösung (500 ppm) mit bzw. ohne 100 ppm (2*S*)-Homoeriodictyol, (*2S*)-Homoeriodictyol-Dinatriumsalz, (2*S*)-Eriodictyol, (2*S*)-Eriodictyol-7-methylether und (2*S*)-Naringenin verkostet und anhand der Referenzreihe eingestuft.

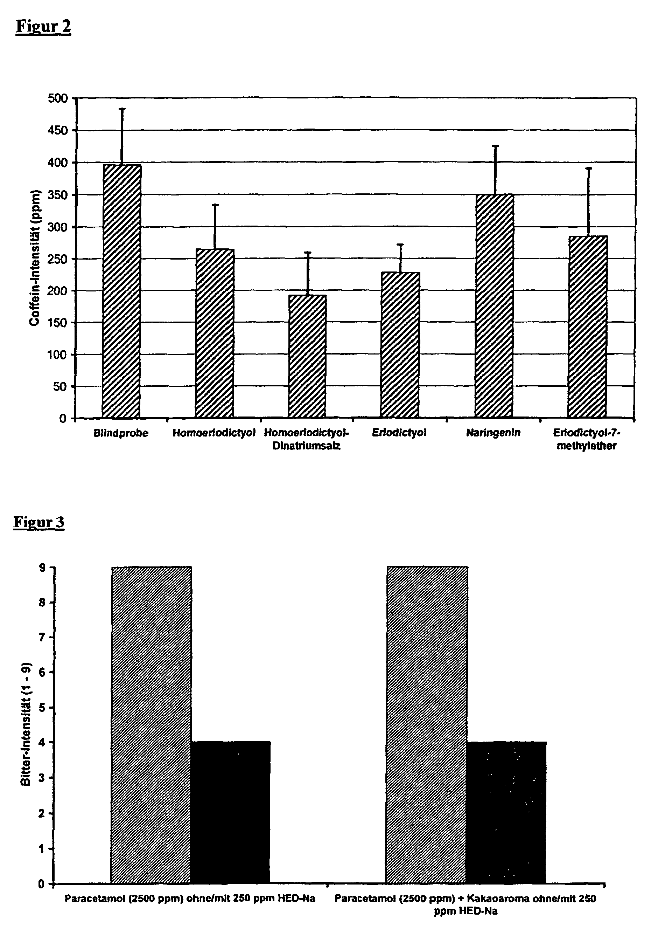

Fig. 2 zeigt die Einschätzung der Coffein-Intensität einer 500 ppm Coffein und 100 ppm eines beispielhaften Hydroxyflavanons enthaltenden Lösung

### Beispiel 5

Die Bittereindrücke einer Paracetamol-Lösung, einer Paracetamol/Homoeriodictyol-Dinatriumsalz und einer Paracetamol/ Homoeriodictyol-Dinatriumsalz und Schokoladenaroma enthaltenden Lösung wurde anhand einer Skala von 1 bis 9 eingestuft.

Fig. 3 zeigt die Abnahme der Bitterintensität für Paracetamol.

## Patentansprüche

1. Verwendung von Verbindungen der allgemeinen Formel (I) 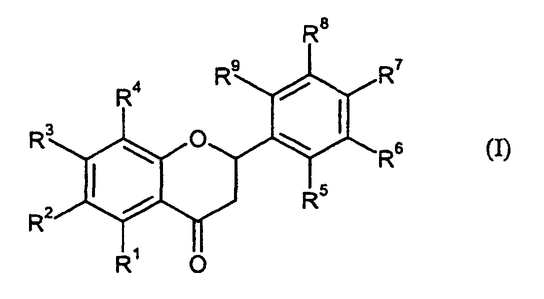 wobei
R², R⁴, R⁵ und R⁹ Wasserstoffatome darstellen und
R¹, R³, R⁶, R⁷ und R⁸ unabhängig voneinander Wasserstoffatome, Hydroxygruppen, Methyl-, Methoxy- oder Ethoxygruppen bedeuten, mit der Maßgabe, dass mindestens einer der Reste R⁶ bis R⁸ eine Hydroxygruppe darstellt.
als Bestandteil von in der Ernährung oder dem Genuss dienenden sowie oralen pharmazeutischen Zubereitungen, enthaltend mindestens einen Bitterstoff oder einen Stoff, der einen bitteren Nachgeschmack verursacht, zur Maskierung oder Verminderung des bitteren oder metallischen Geschmackseindrucks.

2. Verwendung nach Anspruch 1, **dadurch gekennzeichnet, dass** in der Formel (I),
R², R⁴, R⁵, R⁸ und R⁹ Wasserstoffatome darstellen und
R¹, R³ und R⁶ unabhängig voneinander Wasserstoffatome, Hydroxy- oder Methoxygruppen bedeuten, mit der Maßgabe, dass mindestens einer der Reste R¹ und R³ eine Hydroxygruppe darstellt, .
und
R⁷ eine Hydroxygruppe darstellt.

3. Verwendung von 2-(4-Hydroxyphenyl)-5,7-dihydroxychroman-4-on (Naringenin), 2-(3,4-Dihydroxyphenyl)-5,7-dihydroxychroman-4-on (Eriodictyol), 2-(3,4-Dihydroxyphenyl)-5-hydroxy-7-methoxychroman-4-on (Eriodictyol-7-methylether), 2-(3,4-Dihydroxyphenyl)-7-hydroxy-5-methoxychroman-4-on (Eriodictyol-5-methylether) und 2-(4-Hydroxy-3-methoxyphenyl)-5,7-dihydroxychroman-4-on (Homoeriodictyol) als Bestandteil in der Ernährung oder dem Genuss dienenden sowie oralen pharmazeutischen Zubereitungen, enthaltend mindestens einen Bitterstoff oder einen Stoff, der einen bitteren Nachgeschmack verursacht, zur Maskierung oder Verminderung des bitteren oder metallischen Geschmackseindrucks.

4. Verwendung nach einem der Ansprüche 1 bis 3, **dadurch gekennzeichnet, dass** die Verbindungen der Formel (I) als ein- oder, im Falle mehrerer Hydroxygruppen, mehrwertige Anionen vorliegen, wobei als Gegenkationen die einfach positiv geladenen Kationen der ersten Haupt- und Nebengruppe, das Ammoniumion, ein Trialkylammoniumion, die zweiwertig geladenen Kationen der zweiten Haupt- und Nebengruppe sowie die dreiwertigen Kationen der 3. Haupt- und Nebengruppe dienen.

5. Verwendung nach einem der Ansprüche 1 bis 4, **dadurch gekennzeichnet, dass** die Verbindungen der Formel (I) als (2*R*)- oder (2*S*)-Enantiomere oder als Gemisch derselben vorliegen.

6. Der Ernährung oder dem Genuss dienende Zubereitungen, **dadurch gekennzeichnet, dass** sie 0,000001 Gew.-% bis 95 Gew.-%, bezogen auf das Gesamtgewicht der Zubereitung, an Verbindungen der allgemeinen Formel (I) 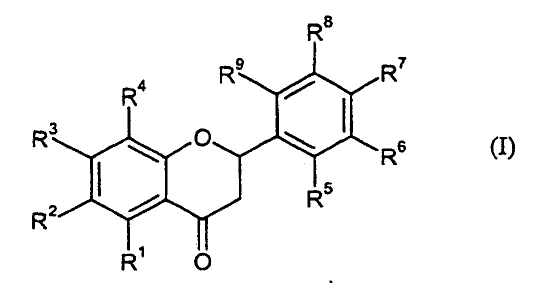 wobei
R², R⁴, R⁵ und R⁹ Wasserstoffatome darstellen und
R¹, R³, R⁶, R⁷ und R⁸ unabhängig voneinander Wasserstoffatome, Hydroxygruppen, Methyl-, Metboxy- oder Ethoxygruppen bedeuten, mit der Maßgabe, dass mindestens einer der Reste R⁶ bis R⁸ eine Hydroxygruppe darstellt.
enthalten.

7. Orale pharmazeutische Zubereitungen, **dadurch gekennzeichnet, dass** sie 0,000001 Gew.-% bis 10 Gew.-%, bezogen auf das Gesamtgewicht der Zubereitung, an Verbindungen der allgemeinen Formel (I) 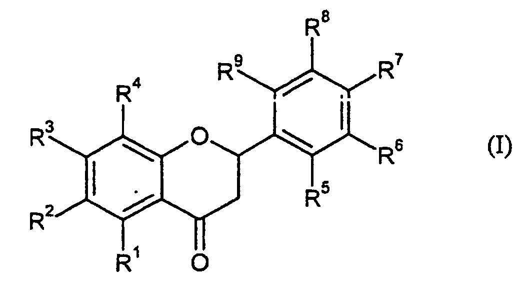 wobei
R², R⁴, R⁵ und R⁹ Wasserstoffatome darstellen und
R¹, R³, R⁶, R⁷ und R⁸ unabhängig voneinander Wasserstoffatome, Hydroxygruppen, Methyl-, Methoxy- oder Ethoxygruppen bedeuten, mit der Maßgabe, dass mindestens einer der Reste R⁶ bis R⁸ eine Hydroxygruppe darstellt.
enthalten.

8. Als Halbfertigwaren vorliegende Zubereitungen nach einem der Ansprüche 6 oder 7.

9. Als Riech-, Aroma- und Geschmacksstoffkompositionen sowie Würzmischungen vorliegende Zubereitungen nach einem der Ansprüche 6 bis 8.

10. Zubereitungen nach einem der Ansprüche 6 bis 9, enthaltend mindestens. einen Bitterstoff.

11. Zubereitungen nach einem der Ansprüche 6 bis 10, enthaltend mindestens einen weiteren bitter-maskierenden Stoff.

12. Zubereitungen nach einem der Ansprüche 6 bis 11, enthaltend eine zusätzliche Aromakomposition.

## Claims

1. Use of compounds of the general formula (I) 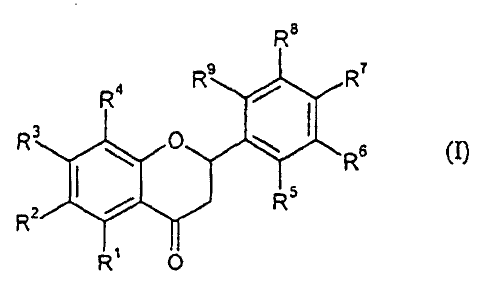 wherein
R², R⁴, R⁵ and R⁹ represent hydrogen atoms and
R¹, R³, R⁶, R⁷ and R⁸ mutually independently mean hydrogen atoms, hydroxy groups, methyl, methoxy or ethoxy groups, with the proviso that at least one of the residues R⁶ to R⁸ represents a hydroxy group,
as a constituent of nutritive preparations or preparations which are otherwise consumed and of oral pharmaceutical preparations, said preparations containing at least one bitter substance or a substance which causes a bitter aftertaste, for masking or reducing the bitter or metallic taste sensation.

2. Use according to claim 1, **characterised in that**, in the formula (I),
R², R⁴, R⁵, R⁸ and R⁹ represent hydrogen atoms and
R¹, R³ and R⁶ mutually independently mean hydrogen atoms, hydroxy or methoxy groups, with the proviso that at least one of the residues R¹ and R³ represents a hydroxy group,
and
R⁷ represents a hydroxy group.

3. Use of 2-(4-hydroxyphenyl)-5,7-dihydroxychroman-4-one (naringenin), 2-(3,4-dihydroxyphenyl)-5,7-dihydroxychroman-4-one (eriodictyol), 2-(3,4-dihydroxyphenyl)-5-hydroxy-7-methoxychroman-4-one (eriodictyol 7-methyl ether), 2-(3,4-dihydroxyphenyl)-7-hydroxy-5-methoxychroman-4-one (eriodictyol 5-methyl ether) and 2-(4-hydroxy-3-methoxyphenyl)-5,7-dihydroxychroman-4-one (homoeriodictyol) as a constituent of nutritive preparations or preparations which are otherwise consumed and of oral pharmaceutical preparations, said preparations containing at least one bitter substance or a substance which causes a bitter aftertaste, for masking or reducing the bitter or metallic taste sensation.

4. Use according to any one of claims 1 to 3, **characterised in that** the compounds of the formula (I) are present as monovalent or, in the case of a plurality of hydroxy groups, polyvalent anions, wherein the counter-cations used are the cations with a single positive charge of main group and subgroup 1, the ammonium ion, a trialkylammonium ion, the cations with a double charge of main group and subgroup 2 and the trivalent cations of main group and subgroup 3.

5. Use according to any one of claims 1 to 4, **characterised in that** the compounds of the formula (I) are present as (2*R*) or (2*S*) enantiomers or as a mixture thereof.

6. Nutritive preparations or preparations which are otherwise consumed, **characterised in that** they contain 0.000001 wt.%. to 95 wt.%, relative to the total weight of the preparation, of compounds of the general formula (I) 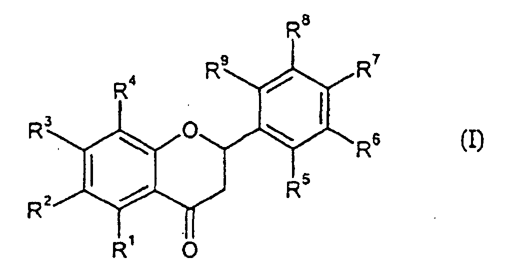 wherein
R², R⁴, R⁵ and R⁹ represent hydrogen atoms and
R¹, R³, R⁶, R⁷ and R⁸ mutually independently mean hydrogen atoms, hydroxy groups, methyl, methoxy or ethoxy groups, with the proviso that at least one of the residues R⁶ to R⁸ represents a hydroxy group.

7. Oral pharmaceutical preparations, **characterised in that** they contain 0.000001 wt.%. to 10 wt.%, relative to the total weight of the preparation, of compounds of the general formula (I) 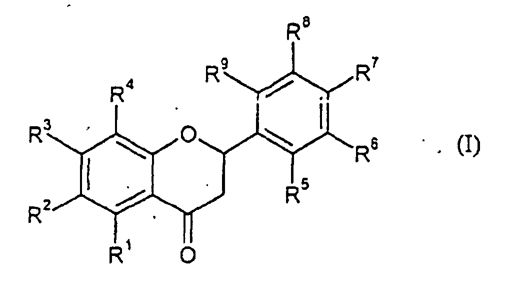 wherein
R², R⁴, R⁵ and R⁹ represent hydrogen atoms and
R¹, R³, R⁶, R⁷ and R⁸ mutually independently mean hydrogen atoms, hydroxy groups, methyl, methoxy or ethoxy groups, with the proviso that at least one of the residues R⁶ to R⁸ represents a hydroxy group.

8. Preparations according to either one of claims 6 or 7 assuming the form of semi-finished products.

9. Preparations according to any one of claims 6 to 8 assuming the form of fragrance, aroma and flavouring compositions and seasoning mixes.

10. Preparations according to any one of claims 6 to 9 containing at least one bitter substance.

11. Preparations according to any one of claims 6 to 10 containing at least one further bitter-masking substance.

12. Preparations according to any one of claims 6 to 11 containing an additional aroma composition.

## Revendications

1. Utilisation de composés de formule générale (I) 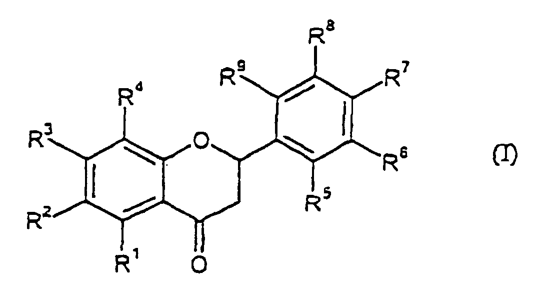 dans laquelle
R², R⁴, R⁵ et R⁹ représentent des atomes d'hydrogène et
R¹, R³, R⁶, R⁷ et R⁸ indépendamment l'un de l'autre, des atomes d'hydrogène, des groupes hydroxy, des groupes méthyle, méthoxy ou éthoxy, étant précisé qu'au moins un des radicaux R⁶ à R⁸ représente un groupe hydroxy,
en tant que constituant de préparations servant à l'alimentation ou à la consommation ainsi que de préparations pharmaceutiques orales, contenant au moins une substance amère ou une substance qui provoque un arrière-goût amer, pour masquer ou réduire l'impression de saveur amère ou métallique.

2. Utilisation selon la revendication 1, **caractérisée en ce que**, dans la formule (I),
R¹, R⁴, R⁵, R⁸ et R⁹ représentent des atomes d'hydrogène et
R¹, R³ et R⁶ indépendamment l'un de l'autre, représentent des atomes d'hydrogène, des groupes hydroxy ou méthoxy, étant précisé qu'au moins un des radicaux R¹ et R³ est un groupe hydroxy,
et
R⁷ représente un groupe hydroxy.

3. Utilisation de 2-(4-hydroxyphényl)-5,7-dihydroxychromane-4-on (naringénine), de 2-(3-4-dihydroxyphényl)-5,7-dihydroxychromane-4-on (ériodictyol), de 2-(3,4-dihydroxyphényl)-5-hydroxy-7-méthoxychromane-4-on (éther 7-méthylique d'ériodictyol), de 2-(3,4-dihydroxyphényl)-7-hydroxy-5-méthoxychromane-4-on (éther 5-méthylique d'ériodictyol) et de 2-(4-hydroxy-3-méthoxyphényl)-5-7-dihyoxychromane-4-on (homoériodictyol) en tant que constituant de préparations servant à l'alimentation ou à la consommation ainsi que de préparations pharmaceutiques orales, contenant au moins une substance amère ou une substance qui provoque un arrière-goût amer, pour masquer ou réduire l'impression de saveur amère ou métallique.

4. Utilisation selon l'une quelconque des revendications 1 à 3, **caractérisée en ce que** les composés de formule (I) se présentent sous forme d'anions monovalents ou, dans le cas de plusieurs groupes hydroxy, polyvalents, les cations à charge simplement positive du premier groupe principal et annexe, l'ion d'ammonium, un ion de trialkylammonium, les cations à charge bivalente, du deuxième groupe principal et annexe ainsi que les cations trivalents du troisième groupe principal et annexe servant de contre-cations.

5. Utilisation selon l'une quelconque des revendications 1 à 4, **caractérisée en ce que** les composés de formule (I) se présentent sous forme d'énantiomères (2*R*) ou (2*S*) ou de mélange de ceux-ci.

6. Préparations servant à l'alimentation ou à la consommation, **caractérisées en ce qu'**elles contiennent 0,000001 % en poids à 95 % en poids, par rapport au poids total de la préparation, de composés de formule générale (I) 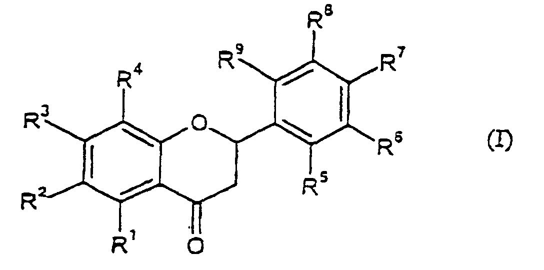 dans laquelle
R², R⁴, R⁵ et R⁹ représentent des atomes de carbone et
R¹, R³, R⁶, R⁷, et R⁸ indépendamment l'un de l'autre, représentent des atomes d'hydrogène, des groupes hydroxy, des groupes méthyle, méthoxy ou éthoxy, étant précisé qu'au moins l'un des radicaux R⁶ à R⁸ est un groupe hydroxy.

7. Préparations pharmaceutiques orales, **caractérisées en ce qu'**elles contiennent 0,000001 % en poids à 10 % en poids, par rapport au poids total de la préparation, de composés de formule générale (I) 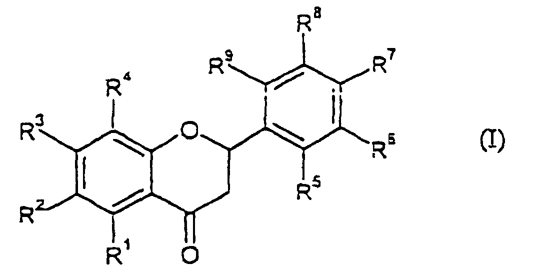 dans laquelle R², R⁴, R⁵ et R⁹ représentent des atomes d'hydrogène et
R¹, R³, R⁶, R⁷ et R⁸ indépendamment l'un de l'autre, représentent des atomes d'hydrogène, des groupes hydroxy, des groupes méthyle, méthoxy ou éthoxy, étant précisé qu'au moins l'un des radicaux R⁶ à R⁸ est un groupe hydroxy.

8. Préparations se présentant sous forme de produits semi-finis selon l'une quelconque des revendications 6 ou 7.

9. Préparations se présentant sous forme de compositions de substances odorantes, aromatiques et sapides ainsi que de mélanges d'épices selon l'une quelconque des revendications 6 à 8.

10. Préparations selon l'une quelconque des revendications 6 à 9, contenant au moins une substance amère.

11. Préparations selon l'une quelconque des revendications 6 à 10 contenant au moins une substance masquant l'amertume.

12. Préparations selon l'une quelconque des revendications 6 à 11, contenant une composition aromatique supplémentaire.
